# EUROPEAN PATENT SPECIFICATION

(11) **EP 0 398 576 B1**
(45) Date of publication and mention of the grant of the patent: **02.08.1995**
(21) Application number: 90304967.4
(22) Date of filing: 09.05.1990
(51) Int. Cl.: C08F 220/00, A61K 7/00, C11D 1/00, B01F 17/00, C09D 133/00, C09J 133/00

(54) **Multi-stage polymer particle having a hydrophobically-modified ionically soluble stage**
Mehrstufiges Polymer-Teilchen mit hydrophobisch-modifiziertem und ionisch-löslichem Polymer-Produktionsabschnitt
Particule de polymère à plusieurs couches comportant une couche de polymére soluble ioniquement modifiée par des groupes hydrophobes

(30) Priority: 15.05.1989 US 352226
(43) Date of publication of application: 22.11.1990
(73) Proprietor: ROHM AND HAAS COMPANY, Philadelphia Pennsylvania 19105 (US)
(72) Inventor: Eisenhart, Eric Karl, Doylestown, PA 18901 (US); Lorah, Dennis Paul, Lansdale, PA 19446 (US); Gill, Susan Renee, Lansdale, PA 19446 (US); Johnson, Eric Alvin, Lansdale, PA 19446 (US)
(74) Representative: Angell, David Whilton

(56) References cited:
- EP-A- 0 011 806
- EP-A- 0 013 836
- EP-A- 0 300 612
- US-A- 4 384 096

## Description

This invention concerns polymer particles which may find use as thickeners. The polymer particles may be useful in a variety of applications such as inks, adhesives, coatings, paints, pigment dispersants, textile thickeners, cosmetic formulations, oil well drilling fluids, liquid detergents and water treatment. The polymer particles of the present invention may exhibit surprising improvements in viscosity stability upon addition of predispersed colorants and upon heat-aging, and improvements in early blister resistance, color float and syneresis resistance.

Many pH-responsive thickeners are known in the art and are used to thicken water-based compositions. These thickeners are generally based upon the incorporation of a hydrophobic surfactant monomer into a hydrophilic, polymeric backbone. The traditional backbone composition for these thickeners primarily included monomeric acid, such as acrylic or methacrylic acid, and an alkyl acrylate or methacrylate, such as ethyl acrylate. The hydrophobic surfactant component is primarily derived from a polyethoxylated alkyl group. Several patents disclose pH-responsive thickeners and the various linkages connecting the hydrophobic surfactant component to the polymer backbone.

US-A-4,384,096 discloses liquid emulsion polymers useful as pH-responsive thickeners containing an acrylate or methacrylate linkage. US-A-4,569,965 discloses crotonate-containing polymeric thickeners. US-A-4,464,524 discloses polymer thickeners containing maleate linkages. US-A-4,663,385 discloses copolymers of alkyl poly(oxyalkylene) itaconic di-esters. US-A-4,616,074 discloses acrylic-methylene succinic ester emulsion copolymers for thickening aqueous systems. US-A-4,338,239 discloses thickener copolymers having allyl glycidyl ether linkages. US-A- 4,514,552 discloses alkali soluble latex thickeners containing urethane linkages. US-A-4,600,761 discloses thickener copolymers containing isocyanato ethyl methacrylate linkages. EP-A-0216479 discloses polymeric thickeners containing allyl ether linkages.

The polymeric thickeners of the prior art have several disadvantages which adversely affect their performance in paint applications. The disadvantages of the prior art thickeners include loss of viscosity upon heat-aging and colorant addition, decreased scrub resistance, and blistering over chalky substrates. It is the object of the present invention to overcome one or more of the disadvantages of the prior art thickeners.

Mixtures or blends of alkali-soluble polymers with alkali-insoluble polymers are known in the art, such as described in US-A-3,037,952. Additionally, EP-A- 0207854 discloses a coating composition containing core-shell polymer particles containing (A) 95-99% by weight of at least one C₁-C₈ alkyl acrylate or methacrylate and (B) 1-5% by weight of at least one water soluble monomer. However, none of these references teach multi-stage polymer particles which are useful as thickeners comprising a hydrophobically-modified, ionically-soluble polymer stage.

In accordance with the present invention, we provide polymer particles each comprising two or more polymer stages wherein:
1) at least one of said polymer stages is an ionically-soluble polymer, said ionically-soluble polymer being polymerized from a monomer mixture comprising;
   a) about 0.1 to about 55% by weight hydrophobic monomer having the formula; 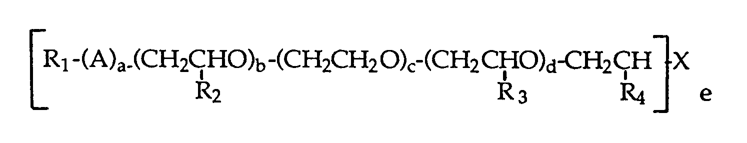 where A is -O-, -S-, 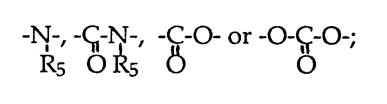 R₁ and R₅ independently are (C₁-C₃₀) alkyl, a (mono-, di-, or tri-) (C₁-C₃₀) alkyl-substituted phenyl ring, or a sorbitan fatty ester; R₂, R₃ and R₄ independently are - H or (C₁-C₁₀) alkyl, aryl, preferably (C₆-C₁₀)aryl, or alkylaryl, preferably (C₁-C₁₀)alkyl (C₆-C₁₀)aryl; a is 0 or 1; b is 0 to 50; c is 0 to 150; d is 0 to 50; e is equal to or greater than 1 and X is a group containing at least one ethylenic double bond;
   b) about 10 to about 60% by weight (C₃-C₃₀) ethylenically-unsaturated, carboxylic acid monomer, and
   c) about 0.1 to about 90% by weight nonionic (C₂-C₃₀) ethylenically-unsaturated monomer having the chemical formula: 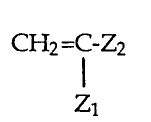
   where Z₁ is -H, -CH₃ or Cl; Z₂ is -CN, -CI, -COOR₈, -C₆H₄R₉ -CHO, 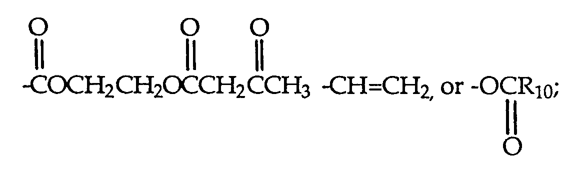 R₈ is C₁-C₁₀ alkyl or C₂-C₈ hydroxyalkyl, R₉ is -H, -Cl, -Br or C₁-C₁₀ alkyl; and R₁₀ is C₁-C₁₀ alkyl, and
d) 0 to about 10% by weight multi-functional compounds;

2) said ionically-soluble polymer is grafted to said polymer particle using one or more multi-functional compounds such that, upon neutralizing said ionically-soluble polymer with base or acid, at least a portion of said ionically-soluble polymer remains attached to the remainder of said polymer particle; and

3) said ionically-soluble polymer comprises from about 1% to about 99% by weight of said polymer particle.

Preferably said ionically-soluble polymer stage comprises from about 50% to about 95%, more preferably about 70% to about 90%, most preferably about 80%, by weight of said polymer particle.

Said ionically-soluble polymer stage may be acid-soluble or base-soluble, but preferably is base-soluble. More preferably the polymer particles of this invention comprise at least one base-soluble polymer stage and at least one base-insoluble polymer stage, wherein the weight ratio of said base-soluble polymer to said base-insoluble polymer is about 99:1 to about 1:99; more preferably about 95:5 to about 50:50. (Similar ratios may apply for acid-soluble polymer: acid-insoluble polymer, polymer particles.)

Each of the polymer stages of the polymer particles of this invention is sequentially polymerized and, as used herein, the term "stage" refers to the polymer formed during each sequence of polymerization. Each stage is also defined as being different from the immediately proceeding and/or immediately subsequent stage by a difference of at least 0.1% by weight in monomer composition. The polymer particles may be prepared by a variety of processes which are well known in the art, such as suspension, emulsion, dispersion, bulk or solution polymerization. Preferably the multi-stage polymer particles of this invention are prepared by emulsion polymerization (reference can be made to US-A-4,427,836 for preferred and specific process conditions). The polymer particles of the present invention are most preferably prepared by sequential emulsion polymerisation.

The polymer particle preferably comprises at least one polymer stage which is base-insoluble and at least one polymer stage which is base-soluble. "Base-insoluble" as used herein means that the polymer is substantially insoluble in aqueous medium which has been adjusted with base to a pH of about 5.0 or greater. The base-insoluble stage has a particle size of 0.1 to about 5000 nanometers.

The composition of the polymer stages, other than said ionically-soluble stage, is not critical and can be of any polymeric composition. Preferably, the polymer particles have at least one base-insoluble polymer stage which is polymerized from a monomer mixture comprising about 1% to about 100% by weight mono-ethylenically-unsaturated monomer and about 0% to about 99% by weight multi-functional compounds (more preferably from about 70 to 99.9% by weight monoethylenically-unsaturated monomers and 0.1% to 30% by weight multi-functional compounds). More preferably, said mono-ethylenically-unsaturated monomers are selected from the group consisting of methyl acrylate, ethyl acrylate, butyl acrylate, 2-ethylhexyl acrylate, decyl acrylate, hydroxyethyl acrylate, hydroxypropyl acrylate, hydroxybutyl acrylate, methyl methacrylate, ethyl methacrylate, butyl methacrylate, hydroxyethyl methacrylate, hydroxypropyl methacrylate, hydroxybutyl methacrylate, acrylonitrile, methacrylonitrile, acrylic acid, methacrylic acid, itaconic acid, maleic acid, fumaric acid, crotonic acid, acrylic anhydride, methacrylic anhydride, maleic anhydride, itaconic anhydride, fumaric anhydride, styrene, substituted styrene, vinyl acetate, vinyl butyrate, vinyl caprolate, acrylamide, methacrylamide, butadiene, isoprene, vinyl chloride, vinylidene chloride, ethylene, propylene and other C₁-C₁₈ alkyl or hydroxyalkyl acrylates, methacrylates, fumarates, maleates or crotonates. The base-insoluble polymer stage can contain from about 0 to about 5% (based on weight of monomer) of chain transfer agents selected from the group consisting of alkyl-mercaptans such as dodecyl mercaptan, t-dodecyl mercaptan, octyl mercaptan, tetradecyl mercaptan, hexadecyl mercaptan and octadecyl mercaptan; hydroxyethyl mercaptan; mercaptopropionic acid; ethyl mercaptopropionate; methyl mercaptopropionate; butyl mercaptopropionate; thioglycolic acid; methyl thioglycolate; ethyl thioglycolate and butyl thioglycolate.

Multi-functional compounds as used herein means a)compounds having two or more sites of unsaturation; b)reactive chain transfer agents having two or more abstractable atoms; c)hybrid compounds having one or more sites of unsaturation and one or more abstractable atoms; d)amine-functional monomers that associate ionically with a base-soluble stage or; e) compounds having one or more sites of unsaturation and one or more nucleophilic or electrophilic reaction sites. Preferably the multi-functional compounds used to polymerize said base-insoluble polymer stage are selected from the group consisting of allyl-, methallyl-, vinyl-, and crotyl-esters of acrylic, methacrylic, maleic (mono-and di-esters), fumaric (mono-and di-esters), and itaconic (mono- and di-esters) acids; allyl-, methallyl-, and crotyl-vinyl ether and thioether; N- and N, N-di-allyl-methallyl-, crotyl-and vinyl-amides of acrylic and methacrylic acids; N-allyl-, methallyl-, and crotyl-maleimide; vinyl esters of 3-butenoic and 4-pentenoic acids; diallyl benzene, diallyl phthalate; triallyl cyanurate; O-allyl-, methallyl-, crotyl-, O-alkyl-, aryl-, P-vinyl-, P-allyl, P-crotyl-, and P-methallyl-phosphonates; triallyl-, trimethallyl-, and tricrotyl-phosphates; O-vinyl-, O, O-diallyl-, dimethallyl-, and dicrotyl-phosphates; cycloalkenyl esters of acrylic, methacrylic, maleic (mono-and di-esters), fumaric (mono- and di-esters), and itaconic (mono- and di-esters) acids [such as dicyclopentenyloxy- ethyl (meth) acrylate and dicyclopentenyl (meth) acrylate]; vinyl ethers and vinyl thioethers of cycloalkenols and cycloalkene thiols; vinyl esters of cycloalkene carboxylic acids; 1,3-butadiene, isoprene and other conjugated dienes; para-methylstyrene; chloromethylstyrene; allyl-, methallyl-, vinyl-, and crotyl-mercaptan; bromotrichloromethane; bromoform; carbon tetrachloride; carbon tetrabromide; N, N'-methylene- bis-acrylamide; ethylene glycol diacrylate; diethylene glycol diacrylate; triethylene glycol diacrylate; tetraethylene glycol diacrylate; polyethylene glycol diacrylate; polypropylene glycol diacrylate; butanediol diacrylate; hexanediol diacrylate; pentaerythritol triacrylate; trimethylolpropane triacrylate; tripropylene glycol diacrylate; neopentyl glycol diacrylate; ethylene glycol dimethacrylate; diethylene glycol dimethacrylate; triethylene glycol dimethacrylate; polyethylene glycol dimethacrylate; polypropylene glycol dimethacrylate; butanediol dimethacrylate; hexanediol dimethacrylate; trimethylolethane trimethacrylate; trimethylolpropane trimethacrylate; divinyl benzene; N, N-dimethylamino ethyl acrylate; N, N-dimethylamino ethyl methacrylate; N, N-diethylamino ethyl acrylate; N, N-diethylamino ethyl methacrylate; N-t-butylamino ethyl acrylate; N- t-butylamino ethyl methacrylate; N, N-dimethylamino propyl acrylamide; N, N-dimethylamino propyl methacrylamide; N, N-diethylamino propyl acrylamide; N, N-diethylamino propyl methacrylamide; p-aminostyrene, N, N-cyclohexylallylamine; 3-N, N-dimethylamino neopentyl acrylate; 3-N, N-dimethylamino neopentyl methacrylate; diallylamine; dimethallylamine; N-ethyl dimethallylamine; N-ethylmethallylamine; N-methyldiallylamine; 2-vinylpyridine; 4-vinyl- pyridine; glycidyl methacrylate; isocyanatoethyl methacrylate; alpha, alpha-dimethyl-m-isopropenyl benzyl isocyanate; chloroethyl acrylate; bromoethyl acrylate; iodoethyl acrylate; chloroethyl methacrylate; bromoethyl methacrylate and iodoethyl methacrylate.

It is critical to the practice of this invention that at least one of the polymer stages of the multi-stage polymer particle be an ionically-soluble polymer. Ionically-soluble means that the polymer is substantially soluble in water when ionized by pH adjustment or chemical reaction (such as quaternization). Ionically-soluble preferably means that the polymer is either acid-soluble or base-soluble as defined herein. The term "acid-soluble" as used herein means that the polymer is substantially soluble in an aqueous medium which has been adjusted with acid to a pH of about 9.0 or less. The term "base-soluble" as used herein means that the polymer is substantially soluble in an aqueous medium which has been adjusted with base to a pH of about 5.0 or greater. The term "ionically-insoluble" as used herein means that the polymer is not ionically-soluble as defined above.
The ionically-soluble polymer stage is polymerized from a monomer mixture comprising
a) 0.1 to 55 parts by weight hydrophobic monomer having the formula; 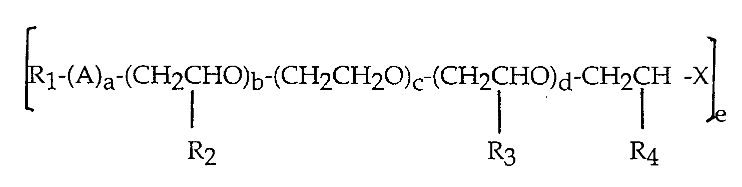 where A is -0-, -S-, 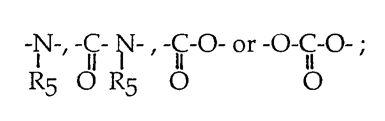 R₁ and R₅ independently are (C₁-C₃₀) alkyl, a (mono-, di-, or tri-)(C₁-C₃₀) alkyl-substituted phenyl ring, or a sorbitan fatty ester; R₂, R₃ and R₄ independently are -H or (C₁-C₁₀) alkyl, aryl or alkylaryl; a is 0 or 1; b is 0 to 50; c is 0 to 150; d is 0 to 50; e is equal to or greater than 1 and X is a group containing at least one ethylenic double bond;
b) 10 to 60 parts by weight (C₃-C₃₀) ethylenically-unsaturated, carboxylic acid monomer and;
c) 0.1 to 90 parts by weight nonionic (C₂-C₃₀) ethylenically-unsaturated monomer having the chemical formula: 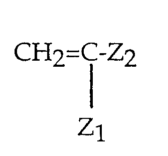 where Z₁ is -H, -CH₃ or Cl; Z₂ is -CN, -Cl, -COOR₈, -C₆H₄R₉, -CHO, 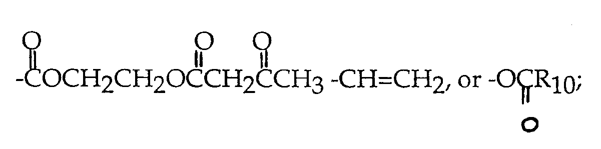 R₈ is C₁-C₁₀ alkyl or C₂-C₈ hydroxyalkyl, R₉ is -H, -Cl, -Br or C₁-C₁₀ alkyl; and R₁₀ is C₁-C₁₀ alkyl, and
d) 0 to 10 parts by weight multi-functional compounds.

Preferably the monomer mixture for preparing the ionically (acid or base)-soluble polymer stage comprises about 2 to about 20% of said hydrophobic monomer. Preparation of hydrophobic monomer which may be used in this invention is described in many literature references, such as US-A- 4075411. In the above formula, X may be any group containing at least one ethylenic double bond, but preferably X is selected from the group consisting of acrylates, methacrylates, crotonates, maleates (mono-and di-esters), fumarates (mono- and di-esters), itaconates (mono-and di-esters), ethylenically-unsaturated urethanes, allyl ethers, methallyl ethers and vinyl ethers.

As previously mentioned, the ionically-soluble polymer stage may be either an acid-soluble polymer or a base-soluble polymer. The acid-soluble polymer stage comprises from 10 to 60 parts by weight (C₃-C₃₀) ethylenically unsaturated, carboxylic acid monomer.

The carboxylic acid monomer used in the polymerization of the base-soluble polymer stage preferably has the chemical formula: 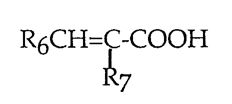
where R₆ is -H, -CH₃ or COOY; R₇ is -H, (C₁-C₄) alkyl or -CH₂COOY; and Y is - H or (C₁-C₁₀) alkyl. Suitable carboxylic acid monomers include, for example, acrylic acid, methacrylic acid, itaconic acid, fumaric acid, maleic acid and crotonic acid. The most preferred carboxylic acid monomer is methacrylic acid.

The nonionic (C₂-C₃₀) ethylenically unsaturated monomers has the chemical formula; 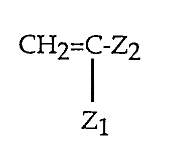
where Z1 is -H, -CH₃ or Cl; Z₂ is -CN, -Cl, -COOR₈,C₆H₄R₉ 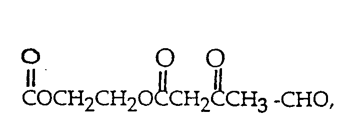
-CH=CH₂, or 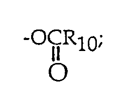
R₈ is C₁-C₁₀ alkyl or C₂-C₈ hydroxyalkyl, R₉ is -H, -Cl, -Br or C₁-C₁₀ alkyl; and R₁₀ is C₁-C₁₀ alkyl.
Examples of suitable nonionic ethylenically-unsaturated monomer are ethyl acrylate, butyl acrylate, methyl methacrylate, butyl methacrylate, 2-ethylhexyl acrylate, 2-hydroxyethyl acrylate, styrene, vinyl acetate, acrylonitrile and vinyl chloride.

The preferred multi-functional compounds which are useful in polymerizing said ionically-soluble polymer are selected from the group consisting of allyl-, methallyl-, vinyl-, and crotyl-esters of acrylic, methacrylic, maleic (mono-and di-esters), fumaric (mono- and di-esters), and itaconic (mono- and di-esters) acids; allyl-,methallyl-, and crotyl-vinyl ether and thioether; N- and N, N-di-allyl, methallyl-, crotyl- and vinyl-amides of acrylic and methacrylic acids; N-allyl-, methallyl-, and crotyl-maleimide; vinyl esters of 3-butenoic and 4-pentenoic acids; diallyl benzene, diallyl phthalate; triallyl cyanurate; O-allyl-, methallyl-, crotyl-, O-alkyl-, aryl-, P-vinyl-, P-allyl, P-crotyl, and P-methallyl-phosphonates; triallyl-, trimethallyl-, and tricrotyl-phosphates; O-vinyl-, O, O-diallyl-, dimethallyl-, and dicrotyl-phosphates; cycloalkenyl esters of acrylic, methacrylic, maleic (mono- and di-esters), fumaric (mono- and di-esters), and itaconic (mono- and di-esters) acids; vinyl ethers and vinyl thioethers of cycloalkenols and cycloalkene thiols; vinyl esters of cycloalkene carboxylic acids; 1, 3-butadiene, isoprene and other conjugated dienes; para-methylstyrene; chloromethylstyrene; allyl-, methallyl-, vinyl-, and crotyl-mercaptan; bromotrichloromethane; bromoform; carbon tetrachloride; carbon tetrabromide; N, N'-methylene- bis-acrylamide; ethylene glycol diacrylate; diethylene glycol diacrylate; triethylene glycol diacrylate; tetraethylene glycol diacrylate; polyethylene glycol diacrylate; polypropylene glycol diacrylate; butanediol diacrylate; hexanediol diacrylate; pentaerythritol triacrylate; trimethylolpropane triacrylate; tripropylene glycol diacrylate; neopentyl glycol diacrylate ethylene glycol dimethacrylate; diethylene glycol dimethacrylate; triethylene glycol dimethacrylate; polyethylene glycol dimethacrylate; polypropylene glycol dimethacrylate; butanediol dimethacrylate; hexanediol dimethacrylate; trimethylolethane trimethacrylate; trimethylolpropane trimethacrylate; divinyl benzene; glycidyl methacrylate; isocyanatoethyl methacrylate; alpha, alpha-dimethyl-m-isopropenyl benzyl isocyanate; chloroethyl acrylate; bromoethyl acrylate; iodoethyl acrylate; chloroethyl methacrylate; bromoethyl methacrylate and iodoethyl methacrylate.

The monomer mixture used to polymerize the ionically-soluble polymer may contain 0 or up to about 5% (based on the weight of said monomer mixture) of chain transfer agents selected from the group consisting of alkyl-mercaptans, such as dodecyl mercaptan, t-dodecyl mercaptan, octyl mercaptan, octyl decyl mercaptan, tetradecyl mercaptan and hexadecyl mercaptan; hydroxyethyl mercaptan; mercaptopropionic acid; methyl mercaptopropionate; ethyl mercaptopropionate; butyl mercaptopropionate; methyl thioglycolate; thioglycolic acid; ethyl thioglycolate and butyl thioglycolate.

In preparing the multi-stage polymer particles of the present invention, the ionically-insoluble polymer stage can be polymerized and subsequently the ionically-soluble polymer stage is polymerized in the presence of the ionically-insoluble polymer stage. Alternatively, the ionically-soluble polymer stage can be polymerized and subsequently the ionically-insoluble polymer stage is polymerized in the presence of the ionically-soluble polymer stage (i.e. inverse polymerization); due to the hydrophobicity of the ionically-insoluble polymer, it becomes one or more domains within the ionically-soluble polymer. A further technique for preparing the multi-stage polymer particles involves polymerization of the ionically-insoluble polymer stage, addition of multi-functional compounds which are allowed to soak into said ionically-insoluble polymer, polymerization of said multi-functional compounds, and subsequent polymerization of said ionically-soluble polymer stage. For example polymer particles comprising base-insoluble polymer and base-soluble polymer may be prepared by emulsion polymerisation. Either said base-insoluble polymer stage is polymerized and subsequently said base-soluble polymer stage is polymerized in the presence of said base-insoluble polymer stage; or said base-soluble polymer stage is polymerized and subsequently said base-insoluble polymer stage is polyermized in the presence of said base-soluble polymer stage and, due to the hydrophobicity of said base-insoluble polymer, it becomes one or more domains within said base-soluble polymer.

The ionically-soluble polymer is physically or chemically attached to the polymer particle such that, upon neutralizing said ionically-soluble polymer (with either a base in the case of a base-soluble polymer or an acid in the case of an acid-soluble polymer) a significant portion (i.e. about 0.5% by weight or greater) of the ionically-soluble polymer remains attached to the remainder of the polymer particle. Physically or chemically attached as used herein means attachment by Van der Waals or London forces, ionic bonding, covalent bonding, hydrogen bonding, chain entanglement or any other means. Preferably the ionically-soluble polymer is chemically grafted to the polymer particle using one or more of the multi-functional compounds described below. Chemical grafting results in permanent attachment of a portion of the ionically-soluble polymer to the polymer particle and results in improved stability toward alcohols/solvents, colorants and other additives.

The following multi-functional compounds are useful to graft said ionically-soluble polymer stage to said ionically-insoluble polymer stage wherein said ionically-insoluble polymer stage is polymerized initially and said ionically-soluble polymer stage is subsequently polymerized: allyl-, methallyl-, vinyl, and crotyl-esters of acrylic, methacrylic, maleic (mono-and di-esters), fumaric (mono- and di-esters), and itaconic (mono- and di-esters) acids; allyl-, methallyl, and crotyl-vinyl ether and thioether; N- and N, N-di-allyl-, methallyl-, crotyl, and vinyl-amides of acrylic and methacrylic acids; N-allyl-, methallyl, and crotyl-maleimide; vinyl esters of 3-butenoic and 4-pentenoic acids; diallyl benzene, diallyl phthalate; triallyl cyanurate; O-allyl-,methallyl-, crotyl-, O-alkyl-, aryl-, P-vinyl-, P-allyl, P-crotyl-, and P-methallyl-phosphonates; triallyl-, trimethallyl-, and tricrotyl-phosphates; O-vinyl-, O, O-diallyl-, dimethallyl-, and dicrotyl-phosphates; cycloalkenyl esters of acrylic, methacrylic, maleic (mono- and di-esters), fumaric (mono- and di-esters), and itaconic (mono- and di-esters) acids; vinyl ethers and vinyl thioethers of cycloalkenols and cycloalkene thiols; vinyl esters of cycloalkene carboxylic acids; 1, 3-butadiene, isoprene and other conjugated dienes; paramethylstyrene; chloromethylstyrene; allyl-, methallyl-, vinyl-, and crotyl-mercaptan; bromotrichloromethane; bromoform; carbon tetrachloride; carbon tetrabromide; glycidyl methacrylate; isocyanatoethel methacrylate; alpha, alpha-dimethyl-m-isopropenyl benzyl isocyanate; chloroethyl acrylate; bromoethyl acrylate; iodoethyl acrylate; chloroethyl methacrylate; bromoethyl methacrylate and iodoethyl methacrylate. The multi-functional compounds listed above are polymerized as part of and during the polymerization of said ionically-insoluble polymer stage.

The following multi-functional compounds are useful for grafting wherein said ionically-insoluble polymer stage is polymerized, followed by the addition of multi-functional compounds which are allowed to soak into said ionically-insoluble polymer, and subsequent sequential polymerization of said multi-functional compounds and said ionically-soluble polymer stage, respectively: N, N-methylene-bis-acrylamide; ethylene glycol diacrylate; diethylene glycol diacrylate; triethylene glycol diacrylate; tetraethylene glycol diacrylate; polyethylene glycol diacrylate; polypropylene glycol diacrylate; butanediol diacrylate; hexanediol diacrylate; pentaerythritol triacrylate; trimethylolpropane triacrylate; tripropylene glycol triacrylate; neopentyl glycol diacrylate; ethylene glycol dimethacrylate; diethylene glycol dimethacrylate; triethylene glycol dimethacrylate; polyethylene glycol dimethacrylate; polypropylene glycol dimethacrylate; butanediol dimethacrylate; hexanediol dimethacrylate; trimethylolethane trimethacrylate; trimethylolpropane trimethacrylate; divinyl benzene; allyl-, methallyl-, vinyl, and crotyl-esters of acrylic, methacrylic, maleic (mono-and di-esters), fumaric (mono- and di-esters), and itaconic (mono- and di-esters) acids; allyl-, methallyl, and crotyl-vinyl ether and thioether; N- and N, N-di-allyl-, methallyl-, crotyl-, and vinyl-amides of acrylic and methacrylic acids; N-allyl-, methallyl, and crotyl-maleimide; vinyl esters of 3-butenoic and 4-pentenoic acids; diallyl benzene, diallyl phthalate; triallyl cyanurate; O-allyl-, methallyl-, crotyl-, O-alkyl-, aryl-, P-vinyl-, P-allyl, P-crotyl-, and P-methallyl-phosphonates; triallyl-, trimethallyl-, and tricrotyl-phosphates; O-vinyl-, O, O-diallyl-, dimethallyl-, and dicrotyl-phosphates; cycloalkenyl esters of acrylic, methacrylic, maleic (mono- and di-esters), fumaric (mono- and di-esters), and itaconic (mono- and di-esters) acids; vinyl ethers and vinyl thioethers of cycloalkenols and cycloalkene thiols; vinyl esters of cycloalkene carboxylic acids; 1, 3-butadiene, isoprene and other conjugated dienes; glycidyl methacrylate; isocyanatoethyl methacrylate; alpha, alpha-dimethyl-m-isopropenyl benzyl isocyanate; chloroethyl acrylate; bromoethyl acrylate; iodoethyl acrylate; chloroethyl methacrylate; bromoethyl methacrylate; iodoethyl methacrylate.

The following multi-functional compounds are useful for grafting wherein said ionically-soluble polymer stage is initially polymerized along with said multi-functional compounds and subsequently said ionically-insoluble polymer stage is polymerized (inverse polymerization): allyl-, methallyl-, vinyl, and crotyl-esters of acrylic, methacrylic, maleic (mono-and di-esters), fumaric (mono- and di-esters), and itaconic (mono- and di-esters) acids; allyl-, methallyl, and crotyl-vinyl ether and thioether; N- and N, N-di-allyl-, methallyl-, crotyl, and vinyl-amides of acrylic and methacrylic acids; N-allyl-, methallyl, and crotyl-maleimide; vinyl esters of 3-butenoic and 4-pentenoic acids; diallyl benzene, diallyl phthalate; triallyl cyanurate; O-allyl-, methallyl-, crotyl-, O-alkyl-, aryl-, P-vinyl-, P-allyl-, P-crotyl-, and P-methallyl-phosphonates; triallyl-, trimethallyl-, and tricrotyl-phosphates; O-vinyl-, O, O-diallyl-, dimethallyl-, and dicrotyl-phosphates; cycloalkenyl esters of acrylic, methacrylic, maleic (mono -and di-esters), fumaric (mon- and di-esters), and itaconic (mono- and di-esters) acids; vinyl ethers and vinyl thioethers of cycloalkenols and cycloalkene thiols; vinyl esters of cycloalkene carboxylic adds; 1, 3-butadiene, isoprene and other conjugated dienes; para-methylstyrene; chloromethylstyrene; allyl-, methallyl-, vinyl-, and crotyl-mercaptan; bromotrichloromethane; bromoform; carbon tetrachloride and carbon tetrabromide: The following multi-functional compounds are useful for grafting wherein said ionically-soluble stage is initially polymerized and subsequently said ionically-insoluble stage is polymerized along with said multi-functional compounds (inverse polymerization): N, N-dimethylamino ethyl acrylate; N, N-dimethylamino ethyl methacrylate; N, N-diethylamino ethyl acrylate; N, N-diethylamino ethyl methacrylate; N-t-butylamino ethyl acrylate; N- t-butylamino ethyl methacrylate; N, N-dimethylamino propyl acrylamide; N, N-dimethylamino propyl methacrylamide; N, N-diethylamino propyl acrylamide; N, N-diethylamino propyl methacrylamide; p-aminostyrene; N, N- cyclohexylallylamine; 3-N, N-dimethylamino neopentyl acrylate; 3-N, N-dimethylamino neopentyl methacrylate; diallylamine; dimethallylamine; N-ethyl dimethallylamine; N-ethylmethallylamine; N-methyldiallylamine; 2-vinylpyridine; 4-vinylpyridine. The multi-functional compounds listed below are useful for grafting wherein said ionically-soluble stage is initially polymerized and subsequently said ionically-insoluble stage is polymerized (inverse polymerization). These multi-functional compounds can be polymerized in either said ionically-soluble or ionically-insoluble stages: glycidyl methacrylate; isocyanatoethyl methacrylate; alpha, alpha-dimethyl-m-isopropenyl benzyl isocyanate; chloroethyl acrylate; bromoethyl acrylate; iodoethyl acrylate; chloroethyl methacrylate; bromoethyl methacrylate; iodoethyl methacrylate.

The most preferred multi-functional compounds for grafting in the inverse polymerization techniques are the crotyl-esters of acrylic, methacrylic, maleic (mono- and di-esters), fumaric (mono- and di-esters), and itaconic (mono- and di-esters) acids (such as crotyl methacrylate); crotyl-vinyl ether and thioether; N-di-crotyl-amides of acrylic and methacrylic acids; N-crotyl-maleimide; O-crotyl-, P-crotyl-phosphonates; tricrotyl-phosphates; dicrotyl-phosphates; cycloalkenyl esters of acrylic, methacrylic, maleic (mono- and di-esters), fumaric (mono- and di-esters), and itaconic (mono- and di-esters) acids (such as dicyclopentenyloxyethylmethacrylate, dicyclopentenyl acrylate, dicyclopentenyl methacrylate); vinyl ethers and vinyl thioethers of cycloalkenols and cycloalkene thiols; vinyl esters of cycloalkene carboxylic acids; and crotyl-mercaptan. The multi-functional compounds listed above are polymerized as part of and during the polymerization of said ionically-soluble polymer stage.

The polymer particles of this invention may be useful in a wide variety of applications as described earlier. The polymer particles may be useful either in dried form or as an emulsion of the polymer particles in an aqueous medium. The polymer particles are preferably used as an aqueous emulsion composition or added to water-containing compositions wherein in either case the ionically-soluble polymer is neutralized and substantially dissolved with either a base or an acid; except that a portion of said ionically-soluble polymer remains atttached or associated with the insoluble polymer stage(s). Based on equivalents of carboxylic acid in the base-soluble polymer, preferably about 0.8 to about 1.5 equivalents of base are introduced to said compositions to neutralize the base-soluble polymer. The neutralized base-soluble polymer is dissolved in the aqueous medium, but a significant portion remains attached to the remainder of the polymer particle.

The base used to neutralize said base-soluble polymer can be any, but is preferably selected from the group consisting of ammonium hydroxide, potassium hydroxide, sodium hydroxide, lithium hydroxide, triethylamine, triethanolamine, monoethanolamine, 2-amino-2-methyl-1-propanol and dimethylaminoethanol.

The polymer particles are useful in a method of thickening water-containing compositions (preferably containing about 20% by weight water or greater) by incorporating the polymer particles therein and neutralizing. The base-soluble polymer is neutralized with base by adjusting the pH of the compositions to about 5.0 or greater. The acid-soluble polymer is neutralized with acid by adjusting the pH of the composition to about 9.0 or less. The amount of the polymer particles used as a thickener depends upon the particular application, but is generally used in an amount of from about 0.1% to about 20% by weight of the total composition. The compositions to be thickened using the polymer particles of this invention can contain many additional ingredients such as pigments, fillers, extenders, surfactants, stabilizers, biocides and the like.

The multistage polymer particles of the present invention may be used in a variety of applications, such as in inks, adhesives, coatings, paints, pigment dispersants, textile thickeners, cosmetic formulations, oil well drilling fluids, liquid detergents and water treatment. Such compositions may contain one or more additional ingredients selected from the group consisting of liquid carriers, e.g. water, pigments, fillers, extenders, surfactants, stabilizers, and biocides.

The following examples are presented to demonstrate this invention. The example are intended in an illustrative, but not limitative, sense. All parts and percentages are by weight unless otherwise indicated.
The following abbreviations are used in the Examples:
- BA: = butyl acrylate
- MMA: = methyl methacrylate
- ALMA: = allyl methacrylate
- MAA: = methacrylic acid
- EA: = ethyl acrylate
- MA-20: = methacrylate ester of a 20 mole ethoxylate of cetyl-stearyl alcohol
- A-103: = disodium ethoxylated nonyl phenol half ester of sulfosuccinic acid
- BMP: = butyl mercaptopropionate
- D.I. water: = deionized water
- CrMA: = crotyl methacrylate
- CPS: = centipoise
- DPH: = diallyl phthalate
- Q-1: = dicyclopentenyloxyethyl methacrylate
- BMA: = butyl methacrylate
- ST: = styrene
- Q-2: = methacrylate ester of a 23 mole ethoxylate of lauryl alcohol
- LMA: = lauryl methacrylate
- TMI-970: = alpha, alpha-dimethyl-m-isopropenyl benzyl isocyanate adduct with 50 mole ethoxylate of nonyl phenol
- Cr-20: = crotyl ester of a 20 mole ethoxylate of cetyl-stearyl alcohol
- Al-20: = allyl ester of a 20 mole ethoxylate of cetyl-stearyl alcohol
- TMI-20: = alpha, alpha-dimethyl-m-isopropenyl benzyl isocyanate adduct with a 20 mole ethoxylate of cetyl-stearyl alcohol.

### Example 1

An emulsion of polymer particles within the scope of this invention was prepared as follows:
A stirred reactor containing 1,532 g. of deionized (D.I.) water, and 9 g. of 28 wt% sodium lauryl sulfate solution (in water) was heated to 80°C. under nitrogen. Next, 42 g. of monomer emulsion (M.E.) #1, shown below, was added to the reactor followed by .95 g. of ammonium persulfate dissolved in 35 g. of D.I. water, and a 24 g. D.I. water rinse. After 10 minutes, the remainder of M.E. #1 and cofeed #1 (shown below) was added to the reactor over a 30-minute period while maintaining the reactor temperature at 80°C. A 47 g. D.I. water rinse was used to flush the feed lines and the water added to the reactor. After a 10 minute hold (at 80°C.), a solution of .45 g. of ammonium persulfate, 1.9 g. of a 33 wt% solution of A-103 (in water), and 70g. of D.I. water was added to the reactor over a 10-minute period. Next M.E. #2 (shown below) and cofeed #2 (shown below) was added to the reactor over a 210-minute time period. The temperature was maintained at 80°C. throughout the additions. At the end of the feeds the monomer emulsion feed lines were flushed with 48 g. of D.I. water and the water added to the reactor. After a 30-minute hold (at 80°C.) the dispersion was cooled.

The final product had a solids content of 32.5%, Brookfield viscosity of 7 centipoises (cps) (0.7 PaS), and a pH of 3.1. When 6.2 g. of this material was mixed with .7 g. of 50 wt% NaOH and 193.1 g of D.I. water the resulting mixture had a viscosity of 102 cps (10.2 PaS) (Brookfield viscometer, 30 rpm). When 12.3 g. of this material was mixed with 1.4 g of 50 wt% NaOH and 186.3 g. of D.I. water the resulting mixture had a Brookfield viscosity of 4,620 cps (462 PaS) (30 rpm).

| | (BASE-INSOLUBLE) STAGE | (BASE-SOLUBLE) STAGE |
|---|---|---|
| | M.E. #1 | M.E. #2 |
| D.I. water | 91.0g | 495.0g |
| Sodium lauryl sulfate (28 wt%) | 10.8g | -- |
| A-103 (33 wt% in water) | -- | 33.3g |
| BA | 142.5g | -- |
| MMA | 95.0g | -- |
| ALMA | 7.5g | -- |
| MAA | 5.0g | 428.0g |
| EA | -- | 500.0g |
| MA-20 (70 wt% solution in MAA) | -- | 71.5g |
| BMP | -- | .6g |

| | COFEED #1 | COFEED #2 |
|---|---|---|
| D.I. water | 35.0 | 150.0g |
| Ammonium Persulfate | .2g | 1.1g |

### EXAMPLE 2A-B

### Ex. 2A

An emulsion of polymer particles within the scope of this invention was prepared as follows:
A stirred reactor containing 650 g. of D.I. water, and 3.8 g. of 28 wt% sodium lauryl sulfate solution (in water) was heated to 83° C. under nitrogen. Next, 18 g. of monomer emulsion (M.E.) #1, shown below, was added to the reactor followed by 0.4 g. of ammonium persulfate dissolved in 15 g. of D.I. water, and a 10 g. D.I. water rinse. After 5 minutes, the remainder of M.E. #1 and cofeed #1 (shown below) was added to the reactor over a 40-minute period while maintaining the reactor temperature at 83° C. A 20 g. D.I. water rinse was used to flush the feed lines and the water added to the reactor. After a 10-minute hold (at 83° C.), the reactor was cooled to 81 °C. and a solution of 0.2 g. of ammonium persulfate, 0.8 g. of a 33 wt% solution of A-103 (in water), and 30 g. of D.I. water was added to the reactor over a 5 minute period. Next M.E. #2 (shown below) and cofeed #2 (shown below) were added to the reactor over a 200 minute time period. The temperature was maintained at 81° C. throughout the additions. At the end of the feeds, the monomer emulsion feed lines were flushed with a 20 g. of D.I. water and the water added to the reactor. After a 30-minute hold (at 81° C.) the dispersion was cooled.

The final product had a solids content of 32.4%, Brookfield viscosity of 11 cps (1.1 PaS), and a pH of 3.0. When 12.4 g. of this material was mixed with 1.4 g. of 50 wt% NaOH and 186.3 g. of D.I. water the resulting mixture had a viscosity of 72 cps (7.2 PaS) (Brookfield viscometer, 30 rpm).

| | M.E. #1 | M.E. #2 |
|---|---|---|
| D.I. Water | 38.5 g. | 195.0 g. |
| Sodium lauryl sulfate (28 wt%) | 4.5 g. | -- |
| A-103 (33 wt% in water) | -- | 14.1 g. |
| BA | 70.5 g. | -- |
| MMA | 30.2 g. | -- |
| MAA | 2.1 g. | 181.9 g. |
| ALMA | 3.2 g. | -- |
| EA | -- | 211.8 g. |
| MA-20 (70 wt% solution in MAA) | -- | 22.7 g. |
| Q-2 (70 wt% solution in MAA) | -- | 7.6 g. |
| BMP | -- | .3 g. |

| | COFEED #1 | COFEED #2 |
|---|---|---|
| D.I. Water | 20.0 g. | 78.0 g. |
| Ammonium Persulfate | .1 g. | .5 g. |

### Ex. 2B

An emulsion of polymer particles within the scope of this invention was prepared as in Ex. 2A above, except that in monomer Emulsion #1 the amount of BA, MMA and ALMA was changed to 62.3 grams, 41.6 grams and 0.0 grams, respectively. The final product had a solids content of 32.0%, Brookfield viscosity of 9 cps (0.9 PaS) and a pH of 3.0. When 3.1 of this material was mixed with 0.7 of 50 wt.% NaOH and 96.3g. of D.I. water, the resulting mixture had a Brookfield (30rpm) viscosity of 380 cps (38 PaS).

### EXAMPLE 3 A-B(Comparative)

Emulsions of polymer particles falling outside the scope of this invention were prepared using conventional emulsion polymerization techniques utilizing the following recipe:

| Example 3A | |
|---|---|
| | Monomer Emulsion |
| D.I. water | 650.0 g. |
| Sodium lauryl sulfate (28% soln) | 35.0 g. |
| EA | 625.0 g. |
| MA-20 (70 wt% solution in MAA) | 89.3 g. |
| MAA | 535.7 g. |
| BMP | .8 g. |

| | COFEED |
|---|---|
| D.I. Water | 150.0 g. |
| Ammonium Persulfate | 1.0 g. |

| Example 3B | |
|---|---|
| | Monomer Emulsion |
| D.I. water | 600.0g. |
| A-103 (33 wt% in water) | 35.2 g. |
| MA-20 (70 wt% solution in MAA) | 56.8 g. |
| Q-2 (70 wt% solution in MAA) | 18.9 g. |
| MAA | 454.3 g. |
| BMP | .7 g. |
| EA | 530.0 g. |

| | COFEED |
|---|---|
| D.I. water | 88.0 g. |
| Ammonium Persulfate | .9 g. |

The final product from Ex. 3A had a solids content of 32.0%, Brookfield (30 rpm) viscosity of 11 cps (1.1 PaS), and a pH of 2.8. The final product from Ex. 3B had a solids content of 33.1%, Brookfield viscosity of 12 cps (1.2 PaS) (30 rpm) and a pH of 3.7.

### EXAMPLE 4 (Comparative)

An emulsion of polymer particles falling outside the scope of this invention was prepared using conventional emulsion polymerization techniques utilizing the following recipe:

| | MONOMER EMULSION |
|---|---|
| D.I. Water | 192.3 g. |
| Sodium lauryl sulfate (28 wt%) | 22.7 g. |
| BA | 302.1 g. |
| MMA | 201.4 g. |
| MAA | 10.6 g. |
| ALMA | 15.9 g. |

| | COFEED |
|---|---|
| D.I. Water | 98.0 g. |
| Ammonium Persulfate | .4 g. |

The final product had a solids content of 39.1%, Brookfield viscosity of 24 cps (2.4 PaS), and a pH of 2.5.

### EXAMPLE 5 (Comparative)

A composition falling outside the scope of this invention was prepared by mixing 1000 grams of the sample prepared in Ex. 3B and 211 grams of the sample prepared in Ex. 4. The resulting mixture had a solids content of 34.4.%, Brookfield viscosity of 13 centipoises (1.3 PaS) and a pH of 3.65. When 5.8 grams of this product was mixed with 1.4 grams of 50 wt.% NaOH and 192.8 grams of D.I. water, the resulting mixture had a viscosity of 58 centipoise (5.8 PaS) (Brookfield viscometer, 30 rpm).

### EXAMPLES 6-10

Various compositions within the scope of this invention were prepared following the procedures of Ex. 1. All of the compositions comprised polymer particles having a base-soluble 2nd stage polymer comprising 50% ethyl acrylate, 45% methacrylic acid, 5% MA-20, and 0.063% butyl mercaptopropionate based on weight of 2nd stage monomers. The compositions of the 1st stage polymer are presented in Table I below. The weight ratio of 2nd stage polymer to 1st stage polymer was 80:20.

**Table I**

| Example | Composition of 1st Stage Polymer(%) | | | |
|---|---|---|---|---|
| | BA | MMA | ALMA | MAA |
| 6 | 58 | 39 | 3 | 0 |
| 7 | 52 | 35 | 3 | 10 |
| 8 | 46 | 31 | 3 | 20 |
| 9 | 28 | 19 | 3 | 50 |
| 10 | 22 | 15 | 3 | 60 |

### EXAMPLES 11-14

Various compositions within the scope of this invention were prepared as in Examples 6-10, except that the 1st stage polymer of the polymer particles had the compositions given in Table II.

**Table II**

| Example | Composition of 1st Stage Polymer (%) | | | |
|---|---|---|---|---|
| | BA | MMA | ALMA | MAA |
| 11 | 59 | 39 | 0 | 2 |
| 12 | 53 | 35 | 10 | 2 |
| 13 | 47 | 31 | 20 | 2 |
| 14 | 41 | 27 | 30 | 2 |

### EXAMPLES 15-23

Various compositions within the scope of this invention were prepared as in Examples 6-10, except that the 1st stage polymer of the polymer particles had the compositions given in Table III.

**Table III**

| Example | Composition of 1st Stage Polymer(%) | | | | | |
|---|---|---|---|---|---|---|
| | BA | MMA | CRMA | Q-1 | DPH | MAA |
| 15 | 58 | 39 | 1 | - | - | 2 |
| 16 | 57 | 38 | 3 | - | - | 2 |
| 17 | 56 | 37 | 5 | - | - | 2 |
| 18 | 53 | 35 | 10 | - | - | 2 |
| 19 | 47 | 31 | 20 | - | - | 2 |
| 20 | 57 | 38 | - | 3 | - | 2 |
| 21 | 53 | 35 | - | 10 | - | 2 |
| 22 | 57 | 38 | - | - | 3 | 2 |
| 23 | 53 | 35 | - | - | 10 | 2 |

### EXAMPLES 24-30

Various compositions within the scope of this invention were prepared following the procedures of Ex. 1. The 2nd stage polymer of the polymer particle comprised 50% ethyl acrylate, 45% methacrylic acid, 5% MA-20 and 0.063% BMP based on weight of 2nd stage monomer. The compositions of the 1st stage polymer are presented in Table IV below. The weight ratio of 2nd stage polymer to 1st stage polymer was 80:20.

**Table IV**

| Example | Composition of 1st Stage Polymer (%) |
|---|---|
| 24 | 95 ethyl hexyl acrylate/3 allyl methacrylate/2 methacrylic acid |
| 25 | 95 styrene/3 allyl methacrylate/2 methacrylic acid |
| 26 | 95 ethyl acrylate/3 allyl methacrylate/2 methacrylic acid |
| 27 | 95 butyl methacrylate/3 allyl methacrylate/2 methacrylic acid |
| 28 | 48 hydroxyethyl methacrylate/47 butyl acrylate/3 allyl methacrylate/2 methacrylic acid |
| 29 | 57.2 butyl acrylate/38.1 methyl methacrylate/3 allyl methacrylate/1.7 acrylic acid |
| 30 | 56.4 butyl acrylate/37.6 methyl methacrylate/3 allyl methacrylate/3.0 itaconic acid |

### EXAMPLES 31-42

Various compositions within the scope of this invention were prepared following the procedures of Ex. 1, except that the 1st stage polymer comprised 57% butyl acrylate, 38% methyl methacrylate, 3% allyl methacrylate and 2% methacrylic acid; and the 2nd stage polymer had compositions as shown in Table V below. The weight ratio of 2nd stage polymer to 1st stage polymer was 80:20. The 2nd stage polymer also contained 0.063% butyl mercaptopropionate, except for Ex. 34 wherein the 2nd stage polymer contained 0.047% methyl mercaptopropionate.

**Table V**

| Example | Composition of 2nd Stage Polymer (%) | | | | | | |
|---|---|---|---|---|---|---|---|
| | EA | MAA | MMA | BA | BMA | ST | MA-20 |
| 31 | 85 | 10 | - | - | - | - | 5 |
| 32 | 75 | 20 | - | - | - | - | 5 |
| 33 | 65 | 30 | - | - | - | - | 5 |
| 34 | 50 | 45 | - | - | - | - | 5 |
| 35 | 35 | 60 | - | - | - | - | 5 |
| 36 | 52 | 45 | - | - | - | - | 3 |
| 37 | 45 | 45 | - | - | - | - | 10 |
| 38 | 15 | 45 | - | - | - | - | 40 |
| 39 | 25 | 45 | 25 | - | - | - | 5 |
| 40 | 25 | 45 | - | 25 | - | - | 5 |
| 41 | 25 | 45 | - | - | 25 | - | 5 |
| 42 | 25 | 45 | - | - | - | 25 | 5 |

### EXAMPLES 43-48

Various compositions within the scope of this invention were prepared following the procedures in Ex. 1, except that the 1st stage polymer comprised 57% butyl acrylate, 38% methyl methacrylate, 3% allyl methacrylate and 2% methacrylic acid; and the 2nd stage polymer had compositions as shown in Table VI below. The 2nd stage polymer also contained 0.063% butyl mercaptopropionate. The weight ratio of 2nd stage polymer to 1st stage polymer was 80:20.

**Table VI**

| Example | Composition of 2nd Stage Polymer (%) | | | | | | | |
|---|---|---|---|---|---|---|---|---|
| | EA | MAA | LMA | Q-2 | TMI-970 | Cr-20 | Al-20 | TMI-20 |
| 44 | 49.7 | 45 | - | 5.3 | - | - | - | - |
| 44 | 44.0 | 45 | - | - | 11 | - | - | - |
| 45 | 53.9 | 45 | 1.1 | - | - | - | - | - |
| 46 | 50 | 45 | - | - | - | 5 | - | - |
| 47 | 50.1 | 45 | - | - | - | - | 4.9 | - |
| 48 | 49.4 | 45 | - | - | - | - | - | 5.6 |

### EXAMPLES 48-54

Compositions within the scope of this invention were prepared following the procedures of Ex. 1, except that the weight ratio of 1st stage polymer to 2nd stage polymer was changed as shown in Table VII below.

**Table VII**

| Example | (weight ratio) | |
|---|---|---|
| | 1st Stage Polymer | 2nd Stage Polymer |
| 49 | 1 | 99 |
| 50 | 5 | 95 |
| 51 | 10 | 90 |
| 52 | 30 | 70 |
| 53 | 70 | 30 |
| 54 | 90 | 10 |

### EXAMPLE 55

An emulsion of polymer particles within the scope of this invention was prepared as follows:
A stirred reactor containing 1,533 g. of D.I. water, and 28 g. of 28 wt% sodium lauryl sulfate solution (in water) was heated to 80°C. under nitrogen. Next, 42 g. of monomer emulsion (M.E.) #1 shown below, was added to the reactor followed by .95 g. of ammonium persulfate dissolved in 35 g. of D.I. water, and a 25 g. D.I. water rinse. After 10 minutes, the remainder of M.E. #1 and cofeed #1 (shown below) was added to the reactor over a 210-minute period while maintaining the reactor temperature at 80°C. A 48 g. D.I. water rinse was used to flush the feed lines and the water added to the reactor. After a 10-minute hold (at 80°C.), a solution of .45 g. of ammonium persulfate, 1.9 g. of a 33 wt% solution of A-103 (in water), and 70 g. of D.I. water was added to the reactor over a 10-minute period. Next M.E. #2 (shown below) and cofeed #2 (shown below) was added to the reactor over a 30-minute time period. The temperature was maintained at 80°C. throughout the additions. At the end of the feeds the monomer emulsion feed lines were flushed with 47 g. of D.I. water and the water added to the reactor. After a 30-minute hold (at 80°C.) the dispersion was cooled.

The final product had a solids content of 33.8%, viscosity of 10 cps (1 PaS), and a pH of 2.5. When 5.9 g. of this material was mixed with .7 g of 50 wt% NaOH and 193.4 g. of D.I. water the resulting mixture had a viscosity of 824 cps (82.4 PaS) (Brookfield viscometer, 30 rpm). When 11.8 g. of this material was mixed with 1.4 g. of 50 wt% NaOH and 186.8 g. of D.I. water the resulting mixture had a Brookfield viscosity of 10,840 cps (1084 PaS) (30 rpm).

| | (BASE-SOLUBLE) STAGE | (BASE-INSOLUBLE) STAGE |
|---|---|---|
| | M.E. #1 | M.E. #2 |
| D.I. water | 495.0g. | 90.0g. |
| Sodium lauryl sulfate (28 wt%) | 28.0g. | 10.8g. |
| EA | 497.5g. | -- |
| MA-20 (70 wt% solution in MAA) | 71.4g. | -- |
| MAA | 428.6g. | -- |
| CrMA | 2.5g. | -- |
| BMP | .6g. | -- |
| BA | -- | 150.0g. |
| MMA | -- | 100.0g. |

| | COFEED #1 | COFEED #2 |
|---|---|---|
| D.I. water | 150.0g. | 48.0g. |
| Ammonium Persulfate | 1.1g. | .2g. |

### EXAMPLE 56

An emulsion of polymer particles within the scope of this invention was prepared as follows:
A 1st stage polymer was prepared following the procedures of Example 1, but using the monomer emulsion [M.E.] #1 described below. Next, the reactor was cooled to 60° C. and 30 g. of 1,3 butylene dimethacrylate added. After stirring for about 10 minutes, solutions of 0.53 g. t-butyl hydroperoxide in 7 g. D.I. water, 0.27 g. sodium sulfoxylate formaldehyde in 8 g. D.I. water, and 2 g. of 0.15 wt% ferrous sulfate heptahydrate were added to the reactor. The temperature rose from 60° to 61° C. After 30 minutes the reactor was heated to 80° C., and a solution of 0.45 g. of ammonium persulfate, 1.9 g. of a 33 wt% solution of A-103 [in water], and 70 g. of D.I. water was added to the reactor over a 10-minute period. Next M.E. #2 [shown below] and cofeed #2 [shown below] was added to the reactor over a 210-minute time period. The temperature was maintained at 80° C. throughout the additions. At the end of the feeds the monomer emulsion feed lines were flushed with 48 g. of D.I. water and the water added to the reactor. After a 30-minute hold at 80° C. the dispersion was cooled.

The final product had a solids content of 32.7% and a Brookfield viscosity of 7 cps (0.7 PaS). When 6.1 g. of this material was mixed with .7 g. of 50 wt% NaOH and 193.2 g. of D.I. water the resulting mixture had a viscosity of 680 cps (68 PaS) (Brookfield viscometer, 30 rpm). When 12.2 g. of this material was mixed with 1.3 g. of 50 wt% NaOH and 186.5 g. of D.I. water the resulting mixture had a Brookfield viscosity of 11,360 cps (1136 PaS)(30 rpm).

| | (BASE-INSOLUBLE) STAGE | (BASE-SOLUBLE) STAGE |
|---|---|---|
| | M.E. #1 | M.E.#2 |
| D.I. water | 91.0 g. | 495.0 g. |
| Sodium lauryl sulfate (28 wt%) | 10.8 g. | -- |
| A-103 (33 wt% in water) | -- | 33.3 g. |
| BA | 147.5 g. | -- |
| MMA | 97.5 g. | -- |
| MAA | 5.0 g. | 428.6 g. |
| EA | -- | 500.0 g. |
| MA-20 (70 wt% solution in MAA) | -- | 71.4 g. |

| | Cofeed #1 | Cofeed #2 |
|---|---|---|
| D.I. water | 48.0 g. | 150.0 g. |
| Ammonium Persulfate | .2 g. | 1.1 g. |

### EXAMPLES 57-60 (Comparative)

Various emulsions of single-stage polymer particles falling outside the scope of this invention were prepared following the procedures of Example 3, but having the following compositions:

| Ex. | COMPOSITION (%) | | | | | | | |
|---|---|---|---|---|---|---|---|---|
| | EA | MAA | MMA | MA-20 | MMP | BMP | CrMA | TMI-970 |
| 57 | 25 | 45 | 25 | 5 | -- | 0.063 | -- | -- |
| 58 | 44 | 45 | -- | -- | -- | 0.063 | -- | 11.0 |
| 59 | 49.75 | 45 | -- | 5 | -- | 0.063 | 0.25 | -- |
| 60 | 50 | 45 | -- | 5 | 0.047 | -- | -- | -- |

### EXAMPLE 61

Emulsions of polymer particles within the scope of this invention were each formulated into a paint composition as a thickener using the following recipe:

| GRIND: | |
|---|---|
| Ingredient | Amount (parts by weight) |
| Propylene glycol | 70.00 |
| Hydrophillic acrylic dispersant | 12.00 |
| Defoamer | 1.0 |
| Water | 45.0 |
| Titanium dioxide | 210.0 |
| Clay | 88.0 |

| Let Down: | |
|---|---|
| Water | 50.0 |
| Acrylic binder | 378.0 |
| Ester-alcohol coalescent | 11.4 |
| Defoamer | 3.0 |
| Biocide | 2.0 |
| Ammonium hydroxide | 1.8 |
| Thickener/water | 201.0 |

Each paint composition was thickened using a sufficient amount of the polymer particles (on a dry weight basis) to give an initial viscosity of about 85 Kreb Units (KU) at a pH of about 9.5 adjusted with ammonium hydroxide. After sitting for a period of 3-5 days, the paint composition was sheared by mixing for 5 minutes and the equilibrium viscosity measured. Comparative compositions were prepared using the same formulation above but incorporating as thickeners the emulsions prepared in comparative examples 3B and 5. The compositions were evaluated for viscosity stability after heat-aging, viscosity stability upon colorant addition and early blister resistance.

The viscosity stability after heat-aging was measured by subjecting approximately 250 grams of the paint composition to storage in a 1/2 pint paint can at 60°C for 10 days. The paint samples were then cooled to room temperature, sheared by mixing for 5 minutes, and viscosity measured. The difference between the viscosity of the heat-aged samples and the equilibrium viscosity is presented in Table VIII below.

Viscosity stability upon colorant addition was evaluated by measuring the viscosity decline which occurred after addition of the equivalent of 8 ounces of pthalo blue colorant to a gallon of each paint composition. This data is presented in Table VIII below.

Early blister resistance was evaluated following ASTM method D659-86 and D-714 by applying two coats of each paint composition to a chalky acrylic substrate with a 6-hour drying time between coats at 77°F (25°C) and 50% relative humidity. After drying overnight, the substrates were subjected to a mist of deionized water and the formation of blister defects were rated over time. The results are presented in Table VIII below (0=worst, 10=best, F=few, M=medium, MD=medium dense, D=dense).

**Table VIII**

| Sample | Thickener/Amount (lbs/gal.) (Kg/l) | Viscosity Stability(KU) | | Early Blister Resistance | | |
|---|---|---|---|---|---|---|
| | | Heat-Aging | Colorant addition | 0.5 hrs. | 1.0 hrs. | 2.0 hrs. |
| A | Ex. 2A 3.53 (0.42) | -7 | -7 | 7D | 6M | 3M |
| B | Ex. 2B 2.82 (0.34) | -9 | -9 | 8F | 6M | 4M |
| C* | Ex. 3B 2.68 (0.32) | -16 | -12 | 7D | 6D | 3D |
| D* | Ex. 5 3.08 (0.37) | -14 | -13 | 7MD | 6D | 6D |

| | | | | | | |
|---|---|---|---|---|---|---|
| *comparative | | | | | | |

The above data demonstrates that samples A and B within the scope of this invention have improved viscosity stability after heat-aging and upon colorant addition when compared to a single-stage thickener (sample C) and a blend of a single-stage thickener and an acrylic emulsion polymer (sample D). The comparative samples C and D showed nearly twice the viscosity loss of samples A and B. The data also shows that samples A and B exhibit equivalent early blister resistance relative to the comparative samples, despite the higher levels of thickener used in Samples A and B. It is generally known in the art that increasing the level of water-sensitive materials such as thickeners can be disadvantageous for early blister resistance.

### EXAMPLE 62

Emulsions of polymer particles within the scope of this invention were formulated into paint compositions and evaluated following the procedures of Example 61. Corresponding comparative formulations were also prepared using emulsions of single-stage polymer particles falling outside the scope of this invention. The results are presented in Table IX.

**Table IX**

| Sample | Thickener/Amount (lbs/gal.) (Kg/l) | Viscosity Stability(KU) | | Early Blister Resistance | | |
|---|---|---|---|---|---|---|
| | | Heat-Aging | Colorant Addition | 0.5 hrs. | 1.0 hrs. | 2.0 hrs. |
| E | Ex. 1/3.47(0.42) | -2 | -8 | 7MD | 6MD | 4MD |
| F* | Ex. 3A/2.00 (0.24) | -16 | -15 | 7MD | 6M | 4MD |
| G | Ex. 39/3.79(0.45) | -8 | -14 | 6D | 5D | 3D |
| H* | Ex. 57/2.14(0.26) | -11 | -14 | 7D | 6D | 5D |
| I | Ex. 34/2.92(0.35) | +1 | -9 | 7MD | 6D | 5D |
| J* | Ex. 60/2.10(0.25) | -14 | -11 | 7MD | 6D | 5D |
| K | Ex. 44/2.98(0.36) | +5 | -11 | 6MD | 5D | 3D |
| L* | Ex. 58/2.10(0.25) | -13 | -14 | 7MD | 6D | 5D |
| M | Ex. 55/2.52(0.30) | +2 | -8 | 8MD | 7MD | 6MD |
| N* | Ex. 59/1.90(0.23) | -12 | -12 | 8MD | 8MD | 7D |

| | | | | | | |
|---|---|---|---|---|---|---|
| *comparative | | | | | | |

### EXAMPLE 63

An emulsion of polymer particles falling within the scope of this invention was prepared as follows:
A stirred reactor containing 600 g. of D.I. water, and 5.2 g. of 42 wt% amphoteric surfactant (Abex-1404, in water) was heated to 60°C. under nitrogen. Then 3.44 g. of 1 wt% versene solution and 3.44 g. of a 0.15 wt% ferrous sulfate heptahydrate solution was added to the reactor. A charge of 16.8 g. of monomer emulsion (M.E.) #1, shown below, was added to the reactor followed by 0.4 g. of ammonium persulfate dissolved in 40 g. of D.I. water, and a 10 g. D.I. water rinse. After 25 minutes, the remainder of M.E. #1, the cofeed initiator #1, and the cofeed reductant #1 (shown below) were added to the reactor over a 210 minute period while maintaining the reactor temperature at 60° C. A 19 g. D.I. water rinse was used to flush the feed lines and the water added to the reactor. After a 30 minute hold (at 60° C.), a solution of 0.12 g. of sodium sulfoxylate formaldehyde in 8 g. of D.I. water was added to the reactor and the temperature increased to 80° C. M.E. #2 (shown below) and cofeed #2 (shown below) were then added to the reactor over a 30 minute time period. The temperature was maintained at 80° C. throughout the additions. At the end of the feeds the mononer emulsion feed lines were flushed with 19 g. of D.I. water and the water added to the reactor. After a 30 minute hold (at 80° C.) the dispersion was cooled to room temperature.

The final product had a solids content of 28.4% and a Brookfield viscosity of 516 cps (51.6 PaS). When 35.2 g. of this material was mixed with 2.5 g. of concentrated hydrochloric acid and 162.3 g. of D.I. water, the resulting mixture had a Brookfield viscosity of 728 cps (72.8 PaS) (30 rpm).

| | M.E. #1 | M.E. #2 |
|---|---|---|
| D.I. water | 198.0 g. | 36.0 g. |
| Amphoteric surfactant (Abex-1404, 42 wt% in water)* | 10.0 g. | 1.16 g. |
| EA | 119.6 g. | -- |
| TMI- | 2020.0 g. | -- |
| Dimethyl aminoethylmethacrylate | 160.0 g. | -- |
| CrMA | 0.4 g. | -- |
| BA | -- | 60.0 g. |
| MMA | 100.0 g. | 40.0 g. |

| | Cofeed initiator #1 | Cofeed #2 |
|---|---|---|
| D.I. water | 80.0 g. | 20.0 g. |
| Ammonium Persulfate | 0.4 g. | .2 g. |

| | Cofeed reductant #1 | |
|---|---|---|
| D.I. water | 40.0 g. | |
| Sodium sulfoxylate formaldehyde | 0.2 g. | |

| | | |
|---|---|---|
| *Abex is a trademark of Alcolac, Inc. | | |

## Claims

1. Polymer particle comprising two or more polymer stages wherein
1) at least one of said polymer stages is an ionically-soluble polymer, said ionically-soluble polymer being polymerized from a monomer mixture comprising:
a) 0.1 to 55 parts by weight based on a), b), c) and d) hydrophobic monomer having the formula; 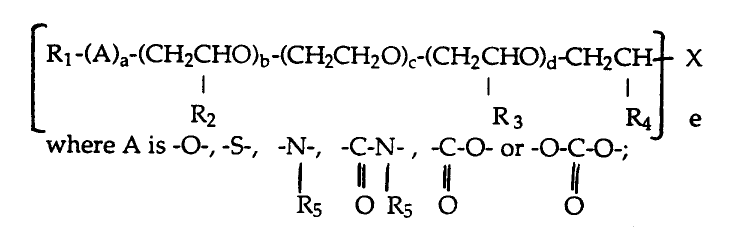 R₁ and R₅ independently are (C₁-C₃₀) alkyl, a(mono-, di-, or tri-)(C₁-C₃₀) alkyl-substituted phenyl ring, or a sorbitan fatty ester; R₂, R₃ and R₄ independently are -H or (C₁-C₁₀) alkyl, aryl or alkylaryl; a is 0 or 1; b is 0 or 1 to 50; c is 0 or 1 to 150; d is 0 or 1 to 50; e is equal to or greater than 1 and X is a group containing at least one ethylenic double bond,
b) 10 to 60 parts by weight based on a), b), c) and d)(C₃-C₃₀), ethylenically-unsaturated, carboxylic acid monomer, and
c) 0.1 to 90% parts by weight based on a), b), c) and d) nonionic (C₂-C₃₀) ethylenically-unsaturated monomer having the chemical formula: 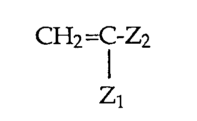 where Z₁ is -H, -CH₃ or Cl; Z₂ is -CN, -CI, -COOR₈, -C₆H₄R₉, - CHO, 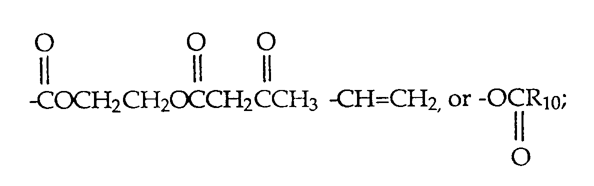 R₈ is C₁-C₁₀ alkyl or C₂-C₈ hydroxyalkyl, R₉ is -H, -Cl, -Br or C₁-C₁₀ alkyl; and R₁₀ is C₁-C₁₀ alkyl., and
d) 0 to 10 parts by weight based on a), b), c) and d) multifunctional compounds;
2) said ionically-soluble polymer is grafted to said polymer particle using one or more multi-functional compounds such that, upon neutralizing said ionically-soluble polymer with base or acid, at least a portion of said ionically-soluble polymer remains attached to the remainder of said polymer particle; and
3) said polymer particle comprises from 1% to 99% by weight of said ionically-soluble polymer when in un-neutralized form or at least 0.5% by weight of said ionically soluble polymer when in neutralized form.

2. Polymer particle as claimed in Claim 1, wherein said ionically-soluble polymer stage is substantially soluble in aqueous medium which has a pH of 5 or greater.

3. Polymer particle as claimed in Claim 2, wherein at least one of said polymer stages is substantially insoluble in aqueous medium which has a pH of 5 or greater, and the weight ratio of said insoluble polymer to said soluble polymer is 1:99 to 99:1.

4. Polymer particle as claimed in anyone of Claims 2-4, wherein said carboxylic acid monomer preferably has the chemical formula: 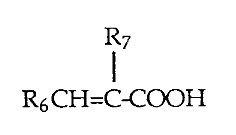 where R₆ is -H, -CH₃, or -COOY; R₇ is -H, (C₁-C₄)alkyl, or -CH₂ COOY; Y is - H or (C₁-C₁₀)alkyl.

5. Polymer particle as claimed in anyone of the preceding Claims, wherein X is selected from the group consisting of acrylates, methacrylates, crotonates, maleates, fumarates, itaconates, ethylenically unsaturated urethanes, allyl ethers, methallyl ethers and vinyl ethers.

6. A composition comprising polymer particles as claimed in any one of the preceding Claims and, optionally, one or more, additional ingredients e.g. liquid carriers, pigments, fillers, extenders, surfactants, stabilizers and biocides.

7. Use of polymer particles as claimed in any one of Claims 1 to 5, or of a composition as claimed in Claim 6, in or as an ink, adhesive, coating, paint, pigment dispersant, textile, thickener, cosmetic formulation, oil well drilling fluid or liquid detergent or in water treatment.

## Patentansprüche

1. Polymerteilchen, umfassend zwei oder mehrere Polymerstufen, wobei
1) mindestens eine der Polymerstufen ein ionisch-lösliches Polymer ist, welches ionisch-lösliche Polymer aus einem Monomergemisch polymerisiert ist, welches umfaßt:
a) 0,1 bis 55 Gewichtsteile, bezogen auf a), b), c) und d), ein hydrophobes Monomer mit der Formel: 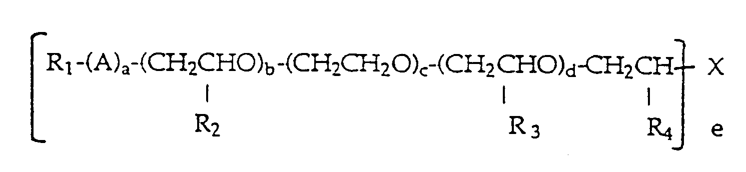 worin A -O-, -S-, 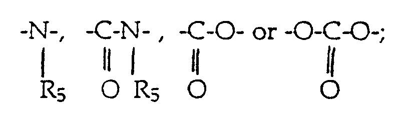 ist;
R₁ und R₅ unabhängig (C₁-C₃₀)Alkyl, ein (Mono-, Di- oder Tri-)(C₁-C₃₀)alkyl-substituierter Phenylring oder ein Sorbitanfettsäureester sind; R₂, R₃ und R₄ unabhängig -H oder (C₁-C₁₀)Alkyl, Aryl oder Alkylaryl sind; a 0 oder 1 ist; b 0 oder 1 bis 50 ist; c 0 oder 1 bis 150 ist; d 0 oder 1 bis 50 ist; e gleich oder größer als 1 ist, und X eine Gruppe ist, welche mindestens eine ethylenische Doppelbindung enthält;
b) 10 bis 60 Gewichtsteile, bezogen auf a), b) c) und d), ein ethylenisch ungesättigtes (C₃-C₃₀)Carbonsäuremonomer; und
c) 0,1 bis 90 Gewichtsteile, bezogen auf a), b), c) und d), ein nichtionisches, ethylenisch ungesättigtes (C₂-C₃₀)Monomer mit der chemischen Formel: 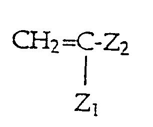 worin Z₁ -H, -CH₃, oder Cl ist; Z₂ -CN, -Cl, -COOR₈, -C₆H₄R₉, -CHO, 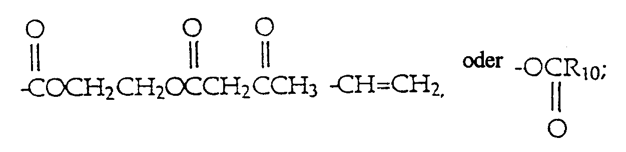 ist;
R₈ (C₁-C₁₀)Alkyl oder (C₂-C₈)Hydroxyalkyl ist, R₉ -H, -Cl, -Br oder (C₁-C₁₀)Alkyl ist; und R₁₀ (C₁-C₁₀)Alkyl ist, und
d) 0 bis 10 Gewichtsteile, bezogen auf a), b), c) und d), multifunktionelle Verbindungen;
2) das ionisch-lösliche Polymer auf das Polymerteilchen gepfropft ist, wobei eine oder mehrere multifuntionelle Verbindungen verwendet wurden, so daß beim Neutralisieren des ionisch-löslichen Polymers mit Base oder Säure mindestens ein Teil des ionisch-löslichen Polymers an den Rest des Polymerteilchens angebracht bleibt; und
3) das Polymerteilchen von 1 Gew.-% bis 99 Gew.-% des ionisch-löslichen Polymers, wenn es in nicht-neutralisierter Form vorliegt, oder mindestens 0,5 Gew.-% des ionisch-löslichen Polymers, wenn es in neutralisierter Form vorliegt, umfaßt.

2. Polymerteilchen, wie in Anspruch 1 beansprucht, wobei die ionisch-lösliche Polymerstufe im wesentlichen in einem wäßrigen Medium, welches einen pH von 5 oder höher aufweist, löslich ist.

3. Polymerteilchen, wie in Anspruch 2 beansprucht, wobei mindestens eine der Polymerstufen im wesentlichen in einem wäßrigen Medium, welches einen pH von 5 oder höher aufweist, unlöslich ist, und wobei das Gewichtsverhältnis des unlöslichen Polymers zum löslichen Polymer 1 : 99 bis 99 : 1 ist.

4. Polymerteilchen, wie in einem der Ansprüche 2 - 4 beansprucht, wobei das Carbonsäuremonomer vorzugsweise die chemische Formel 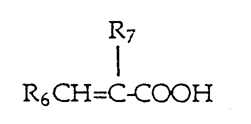 aufweist, worin R₆ -H, -CH₃ oder -COOY ist; R₇ -H, (C₁-C₄)Alkyl oder - CH₂COOY ist; Y -H oder (C₁-C₁₀)Alkyl ist.

5. Polymerteilchen, wie in einem der vorhergehenden Ansprüche beansprucht, wobei X aus der Gruppe von Acrylaten, Methacrylaten, Crotonaten, Maleaten, Fumaraten, Itaconaten, ethylenisch ungesättigten Urethanen, Allylethern, Methallylethern und Vinylethern gewählt ist.

6. Zusammensetzung, umfassend Polymerteilchen, wie in einem der vorhergehenden Ansprüche beansprucht, und gegebenenfalls ein oder mehrere zusätzliche Inhaltsstoffe, z.B. flüssige Träger, Pigmente, Füllstoffe, Streckmittel, Tenside, Stabilisatoren und Biozide.

7. Verwendung von Polymerteilchen, wie in einem der Ansprüche 1 bis 5 beansprucht, oder einer Zusammensetzung, wie in Anspruch 6 beansprucht, in oder als Tinte, Klebstoff, Beschichtung, Anstrich, Anstrichdispergiermittel, Textilmittel, Verdickungsmittel, Kosmetikformulierung, Borflüssigkeit für ein Ölbohrloch oder flüssiges Detergens, oder bei der Wasseraufbereitung.

## Revendications

1. Particule de polymère comprenant deux ou plusieurs couches de polymère dans laquelle
1) au moins une desdites couches de polymère est un polymère ioniquement soluble, ledit polymère ioniquement soluble étant polymérisé à partir d'un mélange monomère comprenant:
a) de 0,1 à 55 parties en poids par rapport à a), b), c) et d) d'un monomère hydrophobe ayant la formule: 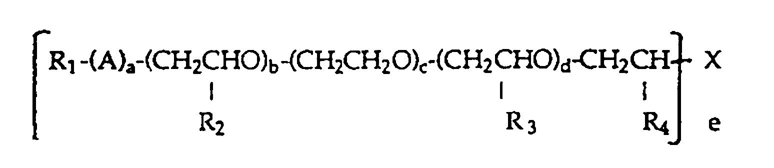 où A est -O-, -S-, 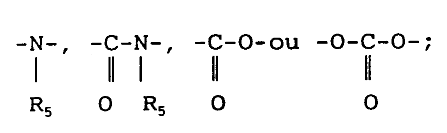 R₁ et R₅ sont indépendamment un groupe alkyle en C₁ à C₃₀, un noyau phényle mono-, di- ou trisubstitué par un groupe alkyle en C₁ à C₃₀, ou un ester gras de sorbitan; R₂, R₃ et R₄ sont indépendamment un atome d'hydrogène ou un groupe alkyle en C₁ à C₁₀, aryle ou alkylaryle; a vaut 0 ou 1; b vaut 0 ou 1 à 50; c vaut 0 ou 1 à 150; d vaut 0 ou 1 à 50; e est égal ou supérieur à 1 et X est un groupe contenant au moins une double liaison éthylénique;
b) de 10 à 60 parties en poids par rapport à a), b), c) et d) d'un monomère acide carboxylique en C₃ à C₃₀ éthyléniquement insaturé, et
c) de 0,1 à 90 parties en poids par rapport à a), b), c) et d) d'un monomère en C₂ à C₃₀ éthyléniquement insaturé non ionique ayant la formule chimique : 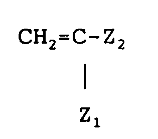 où Z₁ est -H, -CH₃ ou Cl; Z₂ est -CN, -Cl, -COOR₈, -C₆H₄R₉, -CHO, 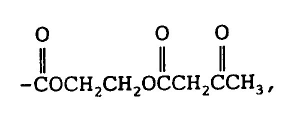 -CH=CH₂ ou 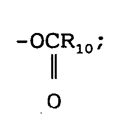 R₈ est un groupe alkyle en C₁ à C₁₀ ou un groupe hydroxyalkyle en C₂ à C₈; R₉ est -H, -Cl, -Br ou un groupe alkyle en C₁ à C₁₀; et R₁₀ est un groupe alkyle en C₁ à C₁₀, et
d) de 0 à 10 parties en poids par rapport à a), b), c) et d) de composés multifonctionnels;
2) ledit polymère ioniquement soluble est greffé à ladite particule de polymère en utilisant un ou plusieurs composés multifonctionnels tels que, après neutralisation dudit polymère ioniquement soluble avec une base ou un acide, au moins une partie dudit polymère ioniquement soluble reste attachée au reste de ladite particule de polymère; et
3) ladite particule de polymère comprend de 1% à 99% en poids dudit polymère ioniquement soluble quand elle est sous forme non neutralisée ou au moins 0,5% en poids dudit polymère ioniquement soluble quand elle est sous forme neutralisée.

2. Particule de polymère selon la revendication 1, dans laquelle ladite couche de polymère ioniquement soluble est sensiblement soluble dans un milieu aqueux qui possède un pH de 5 ou plus.

3. Particule de polymère selon la revendication 2, dans laquelle au moins une desdites couches de polymère est sensiblement insoluble dans un milieu aqueux qui possède un pH de 5 ou plus, et le rapport pondéral dudit polymère insoluble audit polymère soluble est de 1:99 à 99:1.

4. Particule de polymère selon l'une quelconque des revendications 2 à 4, dans laquelle ledit monomère acide carboxylique possède de préférence la formule chimique: 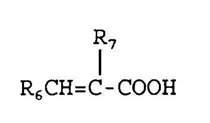 où R₆ est -H, -CH₃ ou -COOY; R₇ est -H, un groupe alkyle en C₁ à C₄ ou -CH₂COOY; Y est -H ou un groupe alkyle en C₁ à C₁₀.

5. Particule de polymère selon l'une quelconque des revendications précédentes, dans laquelle X est choisi parmi le groupe formé par les acrylates, les méthacrylates, les crotonates, les maléates, les fumarates, les itaconates, les uréthanes éthyléniquement insaturés, les éthers allyliques, les éthers méthallyliques et les éthers vinyliques.

6. Composition comprenant des particules de polymère selon l'une quelconque des revendications précédentes, et éventuellement, un ou plusieurs ingrédients supplémentaires, par exemple, des véhicules liquides, des pigments, des charges, des diluants, des tensio-actifs, des stabilisants et des biocides.

7. Utilisation de particules de polymère selon l'une quelconque des revendications 1 à 5, ou d'une composition selon la revendication 6, dans ou comme encre, adhésif, revêtement, peinture, dispersant de pigment, textile, épaississant, formulation cosmétique, fluide de forage de puits de pétrole ou détergent liquide, ou dans le traitement des eaux.
